# EUROPEAN PATENT APPLICATION

(11) **EP 3 357 494 A1**
(43) Date of publication of application: **08.08.2018**
(21) Application number: 16851733.2
(22) Date of filing: 29.09.2016
(51) Int. Cl.: A61K 31/423, A61P 1/08, A61P 1/10, A61P 1/12, A61P 9/00, A61P 19/00, A61P 19/02, A61P 21/00, A61P 21/02, A61P 25/02, A61P 29/00

(54) **THERAPEUTIC AGENT AND/OR PROPHYLACTIC AGENT FOR PERIPHERAL NERVE DISORDER OR SPINAL INJURY**

(30) Priority: 30.09.2015 JP 2015193291
(71) Applicant: National University Corporation Nagoya University, Nagoya-shi, Aichi 464-8601 (JP); Sumitomo Dainippon Pharma Co., Ltd., Osaka-shi Osaka 541-8524 (JP)
(72) Inventor: OHNO, Kinji, Nagoya-shi Aichi 464-8601 (JP); ISHIGURO, Naoki, Nagoya-shi Aichi 464-8601 (JP); OKAWARA, Bisei, Nagoya-shi Aichi 464-8601 (JP); YAGI, Hideki, Nagoya-city, Aichi 453-8511 (JP); OTA, Kyotaro, Nagoya-shi Aichi 464-8601 (JP)
(74) Representative: Vossius & Partner Patentanwälte Rechtsanwälte mbB
(86) International application number: PCT/JP2016/078801
(87) International publication number: WO 2017/057562

(57) **Abstract**

The present invention provides in one embodiment a pharmaceutical composition for treating and/or preventing peripheral neuropathy or spinal cord injury comprising zonisamide or an alkali metal salt thereof as an active ingredient.

## Description

### Technical Field

The present invention relates to a pharmaceutical composition for treating and/or preventing peripheral neuropathy or spinal cord injury comprising zonisamide as an active ingredient.

### Background

Peripheral neuropathy, which is also referred to as neuropathy, is a condition in which normal conduction of peripheral nerve is affected. In peripheral neuropathy, motor nerve, sensory nerve, or autonomic nerve could be impaired. Peripheral neuropathy is clinically classified into mononeuropathy (a disorder affecting a single nerve), multiple mononeuropathy (a disorder affecting two or more nerves in separate areas), or polyneuropathy (an extensive bilateral neuropathy); and pathologically classified into axonopathy, in which axons are mainly damaged, or myelinopathy, in which myelin sheaths degenerate and drop off. Peripheral neuropathy could cause impairment in motor nerve, sensory nerve, and autonomic nerve. This could, in turn, result in symptoms such as pain, paresthesia, paralysis, numbness, muscle weakness, abnormal sweating, or dysuria and will be exacerbated over time. Leading causes of peripheral neuropathy include injury due to a physical factor such as deformation, compression, blood circulation disorder, genetic factors, abnormal metabolism, intoxication, immune-response, infectious disease, cancer, kidney failure, and liver disease. Peripheral neuropathies with unknown causes include facial paralysis.

Methylvitamin B12 (methylcobalamin) is clinically used as a therapeutic agent for peripheral neuropathy (Non Patent Literature 1), although it is rarely effective in clinical practice. Epalrestat, an aldose reductase inhibitor, is used for diabetic peripheral neuropathy (Non Patent Literature 2).

There is still no established method to effectively treat motor paralysis associated with neuropathy, although it seriously affects daily life. Neurotrophins including NGF and BDNF, laminin, and cadherin-11 induce axonal outgrowth of motor neurons and ameliorate movement disorder in animal models (Non Patent Literatures 3-5), but have not been clinically used in human subjects due to their extremely short half-life in a living body (Non Patent Literature 6). Although cell transplantation has been recently focused as a method for treating neuropathy, there remains safety concerns (Non Patent Literatures 7, 8). In addition, although some agents such as nonsteroidal anti-inflammatory agents have been reported to ameliorate motor nerve injuries, there is still no agent that is clinically used for treating motor neuropathy (Non Patent Literature 9).

Spinal cord injury is a condition in which the spinal cord is damaged mainly because the spine is destroyed due to the strong external force applied to the vertebral column. Spinal cord injury could impair motor function, sensory function, or autonomic function. This could, in turn, result in symptoms such as paralysis, pain, paresthesia, phantom limb pain (phantom pain), convulsion, spasticity, contracture, abnormal sweating, abnormal thermoregulation, impaired excretion, or erectile dysfunction. Spinal cord injury could also cause concomitant diseases such as pressure sore, urinary tract infection, and/or pneumonia. Leading causes of spinal cord injury include trauma, injury due to a physical factor such as deformation, compression, blood circulation disorder, and infectious disease.

Once damaged, spinal cord is no longer repaired and regenerated. There is still no established definitive method to recover from spinal cord injury. Although steroids have been reported to suppress subsequent complications of spinal cord injury when administered in a large amount within 48-72 hours after the injury, this procedure is not credible enough and has not been widely used. Also, although a P2X7 receptor antagonist or hepatocyte growth factor (HGF) has been reported to ameliorate spinal cord injury in rats, it has not been used yet to treat spinal cord injury (Non Patent Literatures 10, 11).

Drug repositioning is a strategy to develop novel pharmaceuticals by finding new pharmacological actions of existing pharmaceuticals that have been widely used, or of compounds that have been dropped from development due to insufficient drug efficacy or other reasons. Drug repositioning greatly reduces cost and time required for drug development compared to conventional ways of developing new medicines (Non Patent Literature 12).

Zonisamide (chemical name: 3-sulfamoylmethyl-1,2-benzisoxazole or 1,2-benzisoxazole-3-methanesulfonamide) is a compound with high safety that has been used for 20 years or more from its launching in Japan in 1989. Zonisamide has been used to treat seizures (partial seizure, generalized seizure, and mixed seizure) in children or adults or to treat Parkinson's disease. Zonisamide is useful as an antiepileptic agent or to treat or prevent neurodegenerative diseases such as primary or secondary Parkinson's disease, Huntington's disease, choreatic syndrome, and dystonia syndrome in mammals (including human) (Patent Literatures 1, 2). It has also been reported that zonisamide shows reduction in tardive dyskinesia, anti-obesity effect, and mood-stabilizing effect on bipolar disorder (Non Patent Literatures 13-15) as new pharmacologic actions. In addition, zonisamide has been reported to have neuroprotective effects on dopaminergic neurons or in cerebral ischemia (Non Patent Literatures 16, 17).

These Patent Literatures and Non Patent Literatures, however, do not teach or suggest that zonisamide is effective for peripheral neuropathy or spinal cord injury.

### Citation List

### Patent Literature

Patent Literature 1: Japanese Patent Publication No. 60-03314
Patent Literature 2: Japanese Patent No. 3364481

### Non Patent Literature

Non Patent Literature 1: Yamazaki et al. Neurosci Lett, 170: 195-197 (1994)
Non Patent Literature 2: Ramirez et al. Pharmacotherapy 28: 646-55 (2008)
Non Patent Literature 3: Coumans et al. J Neurosci 21: 9334-9344 (2001)
Non Patent Literature 4: Powell et al. J Biochem Cell Biol 29: 401-414 (1997)
Non Patent Literature 5: Marthiens et al. Mol Cell Neurosci 28: 715-726 (2005)
Non Patent Literature 6: Poduslo et al. Brain Res Mol Brain Res 36: 280-286 (1996)
Non Patent Literature 7: Nakamura et al. Cell Res 23: 70-80 (2013)
Non Patent Literature 8: Guo et al. Histopathology 59: 763-775 (2011)
Non Patent Literature 9: Fu et al. J Neurosci 27: 4154-4164 (2007)
Non Patent Literature 10: Peng et al. Proc Natl Acad Sci 106: 12489-12493 (2009)
Non Patent Literature 11: Peng et al. J Neurosci Res. 85: 2332-2342 (2007)
Non Patent Literature 12: Ashburn et al. Nat Rev Drug Discov 3: 673-683 (2004)
Non Patent Literature 13: Iwata et al. J Neurol Sci. 315: 137-40 (2012)
Non Patent Literature 14: Gadde et al. JAMA. 289(14): 1820-1825 (2003)
Non Patent Literature 15: Hasegawa et al. Curr Med Res Opin. 20(5): 577-80 (2004)
Non Patent Literature 16: Asanuma et al. Ann Neurol 67: 239-249 (2010)
Non Patent Literature 17: Minato et al. Epilepsia 38: 975-980 (1997)

### Summary

The technical problem to be solved by the present invention is to provide a novel use of zonisamide.

The present inventors have intensively studied to solve the problem and found that zonisamide promotes neurite outgrowth and neurite regeneration. It has also been revealed that zonisamide protects neuronal cells from oxidative stress induced by reactive oxygen and increases mRNA expression of nerve growth factors. Furthermore, the present inventors have found that zonisamide administered to model animals with transected sciatic nerve induces nerve regeneration. In addition, the present inventors have found that zonisamide administered to severely crushed spinal cord model animals restores motor function. Then, the present invention has been archieved.

Specifically, the present invention provides the followings:
[1] A pharmaceutical composition for treating and/or preventing peripheral neuropathy comprising zonisamide or an alkali metal salt thereof as an active ingredient.
[2] The pharmaceutical composition according to Item 1, wherein the peripheral neuropathy comprises at least one neuropathy selected from motor neuropathy, sensory neuropathy, or autonomic neuropathy.
[3] The pharmaceutical composition according to Item 2, wherein the peripheral neuropathy at least comprises motor neuropathy.
[4] The pharmaceutical composition according to Item 2 or 3, wherein the motor neuropathy is paralysis, numbness, muscle weakness, hyposthenia, muscle atrophy, disorder of skilled movement, wrist dorsiflexion, ankle dorsiflexion, drop foot, hyporeflexia, or vasomotor symptom.
[5] The pharmaceutical composition according to any one of Items 2-4, wherein the motor neuropathy is paralysis or numbness.
[6] The pharmaceutical composition according to any one of Items 2-5, wherein the sensory neuropathy is pain, paresthesia, or abnormal vibratory sensation.
[7] The pharmaceutical composition according to any one of Items 2-6, wherein the autonomic neuropathy is abnormal sweating, diarrhea, constipation, dizziness, shivering, palpitation, or abnormal heart rate variability.
[8] Use of zonisamide or an alkali metal salt thereof for the manufacture of a medicament for the treatment and/or prevention of peripheral neuropathy.
[9] The use according to Item 8, wherein the peripheral neuropathy comprises at least one neuropathy selected from motor neuropathy, sensory neuropathy, or autonomic neuropathy.
[10] The use according to Item 9, wherein the peripheral neuropathy at least comprises motor neuropathy.
[11] The use according to Item 8 or 9, wherein the motor neuropathy is paralysis, numbness, muscle weakness, hyposthenia, muscle atrophy, disorder of skilled movement, wrist dorsiflexion, ankle dorsiflexion, drop foot, hyporeflexia, or vasomotor symptom.
[12] The use according to any one of Items 9-11, wherein the motor neuropathy is paralysis or numbness.
[13] The use according to any one of Items 9-12, wherein the sensory neuropathy is pain, paresthesia, or abnormal vibratory sensation.
[14] The use according to any one of Items 9-13, wherein the autonomic neuropathy is abnormal sweating, diarrhea, constipation, dizziness, shivering, palpitation, or abnormal heart rate variability.
[15] Zonisamide or an alkali metal salt thereof for use in the treatment and/or prevention of peripheral neuropathy.
[16] Zonisamide or an alkali metal salt thereof according to Item 15, wherein the peripheral neuropathy comprises at least one neuropathy selected from motor neuropathy, sensory neuropathy, or autonomic neuropathy.
[17] Zonisamide or an alkali metal salt thereof according to Item 16, wherein the peripheral neuropathy at least comprises motor neuropathy.
[18] Zonisamide or an alkali metal salt thereof according to Item 16 or 17, wherein the motor neuropathy is paralysis, numbness, muscle weakness, hyposthenia, muscle atrophy, disorder of skilled movement, wrist dorsiflexion, ankle dorsiflexion, drop foot, hyporeflexia, or vasomotor symptom.
[19] Zonisamide or an alkali metal salt thereof according to any one of Items 16-18, wherein the motor neuropathy is paralysis or numbness.
[20] Zonisamide or an alkali metal salt thereof according to any one of Items 16-19, wherein the sensory neuropathy is pain, paresthesia, or abnormal vibratory sensation.
[21] Zonisamide or an alkali metal salt thereof according to any one of Items 16-20, wherein the autonomic neuropathy is abnormal sweating, diarrhea, constipation, dizziness, shivering, palpitation, or abnormal heart rate variability.
[22] A method for treating and/or preventing peripheral neuropathy, comprising administering zonisamide or an alkali metal salt thereof to a patient in need thereof.
[23] The method according to Item 22, wherein the peripheral neuropathy comprises at least one neuropathy selected from motor neuropathy, sensory neuropathy, or autonomic neuropathy.
[24] The method according to Item 23, wherein the peripheral neuropathy at least comprises motor neuropathy.
[25] The method according to Item 23 or 24, wherein the motor neuropathy is paralysis, numbness, muscle weakness, hyposthenia, muscle atrophy, disorder of skilled movement, wrist dorsiflexion, ankle dorsiflexion, drop foot, hyporeflexia, or vasomotor symptom.
[26] The method according to any one of Items 23-25, wherein the motor neuropathy is paralysis or numbness.
[27] The method according to any one of Items 23-26, wherein the sensory neuropathy is pain, paresthesia, or abnormal vibratory sensation.
[28] The method according to any one of Items 23-27, wherein the autonomic neuropathy is abnormal sweating, diarrhea, constipation, dizziness, shivering, palpitation, or abnormal heart rate variability.
[29] The pharmaceutical composition according to any one of Items 1-7, wherein the peripheral neuropathy is selected from the group consisting of Charcot-Marie-Tooth disease, hereditary neuropathy with liability to pressure palsy, hereditary neuralgic amyotrophy, metachromatic leukodystrophy (MLD), Krabbe disease, Fabry disease, adrenoleukodystrophy (ALD), Refsum disease, Tangier disease, acute porphyria, familial amyloid polyneuropathy, Guillain-Barre syndrome, chronic inflammatory demyelinating polyradiculoneuropathy (CIDP), neuropathy associated with AL amyloidosis, diabetic neuropathy, hypothyroidism, neuropathy associated with collagen disease (such as an auto-immune disease and systemic lupus erythematosus), uremic neuropathy, neuropathy associated with chronic liver disease, Crow-Fukase syndrome, Fisher syndrome, critical illness polyneuropathy, neuropathy associated with human immunodeficiency virus, neuropathy associated with varicella-zoster virus, facial paralysis, congenital myasthenic syndrome, myasthenia gravis, sarcopenia, botulism, Eaton Lambert syndrome, drug-induced neuromuscular junction disorder, carpal tunnel syndrome, cubital tunnel syndrome, Guyon tunnel syndrome, musculospiral paralysis, meralgia paresthetica, common fibular nerve paralysis, thoracic outlet syndrome, and tarsal tunnel syndrome.
[30] The pharmaceutical composition according to Item 29, wherein the peripheral neuropathy is selected from the group consisting of Charcot-Marie-Tooth disease, chronic inflammatory demyelinating polyradiculoneuropathy (CIDP), and carpal tunnel syndrome.
[31] The use according to any one of Items 8-14, wherein the peripheral neuropathy is selected from the group consisting of Charcot-Marie-Tooth disease, hereditary neuropathy with liability to pressure palsy, hereditary neuralgic amyotrophy, metachromatic leukodystrophy (MLD), Krabbe disease, Fabry disease, adrenoleukodystrophy (ALD), Refsum disease, Tangier disease, acute porphyria, familial amyloid polyneuropathy, Guillain-Barre syndrome, chronic inflammatory demyelinating polyradiculoneuropathy (CIDP), neuropathy associated with AL amyloidosis, diabetic neuropathy, hypothyroidism, neuropathy associated with collagen disease (such as an auto-immune disease and systemic lupus erythematosus), uremic neuropathy, neuropathy associated with chronic liver disease, Crow-Fukase syndrome, Fisher syndrome, critical illness polyneuropathy, neuropathy associated with human immunodeficiency virus, neuropathy associated with varicella-zoster virus, facial paralysis, congenital myasthenic syndrome, myasthenia gravis, sarcopenia, botulism, Eaton Lambert syndrome, drug-induced neuromuscular junction disorder, carpal tunnel syndrome, cubital tunnel syndrome, Guyon tunnel syndrome, musculospiral paralysis, meralgia paresthetica, common fibular nerve paralysis, thoracic outlet syndrome, and tarsal tunnel syndrome.
[32] The use according to Item 31, wherein the peripheral neuropathy is selected from the group consisting of Charcot-Marie-Tooth disease, chronic inflammatory demyelinating polyradiculoneuropathy (CIDP), and carpal tunnel syndrome.
[33] Zonisamide or an alkali metal salt thereof according to any one of Items 15-21, wherein the peripheral neuropathy is selected from the group consisting of Charcot-Marie-Tooth disease, hereditary neuropathy with liability to pressure palsy, hereditary neuralgic amyotrophy, metachromatic leukodystrophy (MLD), Krabbe disease, Fabry disease, adrenoleukodystrophy (ALD), Refsum disease, Tangier disease, acute porphyria, familial amyloid polyneuropathy, Guillain-Barre syndrome, chronic inflammatory demyelinating polyradiculoneuropathy (CIDP), neuropathy associated with AL amyloidosis, diabetic neuropathy, hypothyroidism, neuropathy associated with collagen disease (such as an auto-immune disease and systemic lupus erythematosus), uremic neuropathy, neuropathy associated with chronic liver disease, Crow-Fukase syndrome, Fisher syndrome, critical illness polyneuropathy, neuropathy associated with human immunodeficiency virus, neuropathy associated with varicella-zoster virus, facial paralysis, congenital myasthenic syndrome, myasthenia gravis, sarcopenia, botulism, Eaton Lambert syndrome, drug-induced neuromuscular junction disorder, carpal tunnel syndrome, cubital tunnel syndrome, Guyon tunnel syndrome, musculospiral paralysis, meralgia paresthetica, common fibular nerve paralysis, thoracic outlet syndrome, and tarsal tunnel syndrome.
[34] Zonisamide or an alkali metal salt thereof according to Item 33, wherein the peripheral neuropathy is selected from the group consisting of Charcot-Marie-Tooth disease, chronic inflammatory demyelinating polyradiculoneuropathy (CIDP), and carpal tunnel syndrome.
[35] The method according to any one of Items 22-28, wherein the peripheral neuropathy is selected from the group consisting of Charcot-Marie-Tooth disease, hereditary neuropathy with liability to pressure palsy, hereditary neuralgic amyotrophy, metachromatic leukodystrophy (MLD), Krabbe disease, Fabry disease, adrenoleukodystrophy (ALD), Refsum disease, Tangier disease, acute porphyria, familial amyloid polyneuropathy, Guillain-Barre syndrome, chronic inflammatory demyelinating polyradiculoneuropathy (CIDP), neuropathy associated with AL amyloidosis, diabetic neuropathy, hypothyroidism, neuropathy associated with collagen disease (such as an auto-immune disease and systemic lupus erythematosus), uremic neuropathy, neuropathy associated with chronic liver disease, Crow-Fukase syndrome, Fisher syndrome, critical illness polyneuropathy, neuropathy associated with human immunodeficiency virus, neuropathy associated with varicella-zoster virus, facial paralysis, congenital myasthenic syndrome, myasthenia gravis, sarcopenia, botulism, Eaton Lambert syndrome, drug-induced neuromuscular junction disorder, carpal tunnel syndrome, cubital tunnel syndrome, Guyon tunnel syndrome, musculospiral paralysis, meralgia paresthetica, common fibular nerve paralysis, thoracic outlet syndrome, and tarsal tunnel syndrome.
[36] The method according to Item 35, wherein the peripheral neuropathy is selected from the group consisting of Charcot-Marie-Tooth disease, chronic inflammatory demyelinating polyradiculoneuropathy (CIDP), and carpal tunnel syndrome.
[37] A pharmaceutical composition for treating and/or preventing spinal cord injury comprising zonisamide or an alkali metal salt thereof as an active ingredient.
[38] The pharmaceutical composition according to Item 37, wherein the spinal cord injury is associated with at least one dysfunction selected from motor dysfunction, sensory dysfunction, and autonomic dysfunction.
[39] The pharmaceutical composition according to Item 38, wherein the spinal cord injury is at least associated with motor dysfunction.
[40] The pharmaceutical composition according to Item 37, wherein the pharmaceutical composition is for treating and/or preventing motor dysfunction, sensory dysfunction, or autonomic dysfunction associated with spinal cord injury.
[41] The pharmaceutical composition according to Item 40, wherein the pharmaceutical composition is for treating and/or preventing motor dysfunction associated with spinal cord injury.
[42] The pharmaceutical composition according to any one of Items 38-41, wherein the motor dysfunction is paralysis, numbness, muscle weakness, hyposthenia, disorder of skilled movement, wrist dorsiflexion, ankle dorsiflexion, drop foot, hyporeflexia, vasomotor symptom, convulsion, spasticity, or contracture.
[43] The pharmaceutical composition according to any one of Items 38-42, wherein the motor dysfunction is paralysis or numbness.
[44] The pharmaceutical composition according to any one of Items 38-43, wherein the sensory dysfunction is pain, paresthesia, or phantom limb pain.
[45] The pharmaceutical composition according to any one of Items 38-44, wherein the autonomic dysfunction is abnormal sweating, diarrhea, constipation, dizziness, shivering, palpitation, abnormal heart rate variability, or impaired excretion.
[46] Use of zonisamide or an alkali metal salt thereof for the manufacture of a medicament for the treatment and/or prevention of spinal cord injury.
[47] The use according to Item 46, wherein the spinal cord injury is associated with at least one dysfunction selected from motor dysfunction, sensory dysfunction, -and autonomic dysfunction.
[48] The use according to Item 45, wherein the spinal cord injury is at least associated with motor dysfunction.
[49] The use according to Item 46, wherein the medicament is for the treatment and/or prevention of motor dysfunction, sensory dysfunction, or autonomic dysfunction associated with spinal cord injury.
[50] The use according to Item 46, wherein the medicament is for the treatment and/or prevention of motor dysfunction associated with spinal cord injury.
[51] The use according to any one of Items 47-50, wherein the motor dysfunction is paralysis, numbness, muscle weakness, hyposthenia, disorder of skilled movement, wrist dorsiflexion, ankle dorsiflexion, drop foot, hyporeflexia, vasomotor symptom, convulsion, spasticity, or contracture.
[52] The use according to any one of Items 47-51, wherein the motor dysfunction is paralysis or numbness.
[53] The use according to any one of Items 47-52, wherein the sensory dysfunction is pain, paresthesia, or phantom limb pain.
[54] The use according to any one of Items 47-53, wherein the autonomic dysfunction is abnormal sweating, diarrhea, constipation, dizziness, shivering, palpitation, abnormal heart rate variability, or impaired excretion.
[55] Zonisamide or an alkali metal salt thereof for use in the treatment and/or prevention of spinal cord injury.
[56] Zonisamide or an alkali metal salt thereof according to Item 55, wherein the spinal cord injury is associated with at least one dysfunction selected from motor dysfunction, sensory dysfunction, and autonomic dysfunction.
[57] Zonisamide or an alkali metal salt thereof according to Item 56, wherein the spinal cord injury is at least associated with motor dysfunction.
[58] Zonisamide or an alkali metal salt thereof according to Item 55, wherein zonisamide or an alkali metal salt thereof is for use in the treatment and/or prevention of motor dysfunction, sensory dysfunction, or autonomic dysfunction associated with spinal cord injury.
[59] Zonisamide or an alkali metal salt thereof according to Item 58, wherein zonisamide or an alkali metal salt thereof is for use in the treatment and/or prevention of motor dysfunction associated with spinal cord injury.
[60] Zonisamide or an alkali metal salt thereof according to any one of Items 56-59, wherein the motor dysfunction is paralysis, numbness, muscle weakness, hyposthenia, disorder of skilled movement, wrist dorsiflexion, ankle dorsiflexion, drop foot, hyporeflexia, vasomotor symptom, convulsion, spasticity, or contracture.
[61] Zonisamide or an alkali metal salt thereof according to any one of Items 56-60, wherein the motor dysfunction is paralysis or numbness.
[62] Zonisamide or an alkali metal salt thereof according to any one of Items 556-61, wherein the sensory dysfunction is pain, paresthesia, or phantom limb pain.
[63] Zonisamide or an alkali metal salt thereof according to any one of Items 56-62, wherein the autonomic dysfunction is abnormal sweating, diarrhea, constipation, dizziness, shivering, palpitation, abnormal heart rate variability, or impaired excretion.
[64] A method for treating and/or preventing spinal cord injury, comprising administering zonisamide or an alkali metal salt thereof to a patient in need thereof.
[65] The method according to Item 64, wherein the spinal cord injury is associated with at least one neuropathy selected from motor dysfunction, sensory dysfunction, and autonomic dysfunction.
[66] The method according to Item 65, wherein the spinal cord injury is at least associated with motor dysfunction.
[67] The method according to Item 64, wherein the method is for treating and/or preventing motor dysfunction, sensory dysfunction, or autonomic dysfunction associated with spinal cord injury.
[68] The method according to Item 67, wherein the method is for treating and/or preventing motor dysfunction associated with spinal cord injury.
[69] The method according to any one of Items 65-68, wherein the motor dysfunction is paralysis, numbness, muscle weakness, hyposthenia, disorder of skilled movement, wrist dorsiflexion, ankle dorsiflexion, drop foot, hyporeflexia, vasomotor symptom, convulsion, spasticity, or contracture.
[70] The method according to any one of Items 65-69, wherein the motor dysfunction is paralysis or numbness.
[71] The method according to any one of Items 65-70, wherein the sensory dysfunction is pain, paresthesia, or phantom limb pain.
[72] The method according to any one of Items 65-71, wherein the autonomic neuropathy is abnormal sweating, diarrhea, constipation, dizziness, shivering, palpitation, abnormal heart rate variability, or impaired excretion.

Zonisamide or a pharmaceutically acceptable salt thereof of the present invention is a compound with high safety and effective in the treatment and/or prevention of peripheral neuropathy or spinal cord injury, in particular peripheral neuropathy or spinal cord injury caused by trauma.

### Brief Description of Drawings

Figure 1 shows dose-dependent effects of zonisamide on average neurite length of NSC34 cells or primary motor neurons, the number of branch points per neuron in primary motor neurons, and ratio of neurite-bearing cells in primary motor neurons.
Figure 2A shows photographs for comparing neurites treated with and without zonisamide in scratch assay.
Figure 2B shows a dose-dependent effect of zonisamide on sum of neurite lengths in scratch assay.
Figure 3 shows dose- and time-dependent effects of zonisamide on neurite outgrowth of mouse primary motor neurons, as measured by time-lapse imaging.
Figure 4 shows neuroprotective effect of zonisamide on cell death caused by oxidative stress in primary motor neurons.
Figure 5 shows mRNA expression levels of Bdnf, Ngf, Ntf4, Ntrk1, Ntrk2, Map2, Mapt, and Gap43 in primary motor neurons treated with zonisamide.
Figure 6 shows phosphorylation of Erk1/2 and JNK1/2/3 measured in primary motor neurons treated with zonisamide.
Figure 7 shows photographs of toluidine blue stained-cross sections that were transversally cut at the position 0.7 mm distal to the distal transection site one week after surgery in the mouse sciatic nerve autologous transplantation model.
Figure 8 shows the total number and the area of axons per nerve fiber in the cross sections that were transversally cut at the position 0.7 mm distal to the distal transection site one week after surgery in the mouse sciatic nerve autologous transplantation model.
Figure 9 shows the effect of zonisamide to improve motor function in the mouse sciatic nerve autologous transplantation model.
Figure 10 shows photographs of hematoxylin and eosin-stained cross sections of tibialis anterior muscle 8 weeks after surgery in the mouse sciatic nerve autologous transplantation model.
Figure 11 shows nerve regeneration with zonisamide in cross-sectional area (CSA) of tibialis anterior muscle fibers 8 weeks after surgery in the mouse sciatic nerve autologous transplantation model.
Figure 12 shows mRNA expression levels of Chrne, Colq, and Rapsn in gastrocnemius muscle 8 weeks after surgery in the mouse sciatic nerve autologous transplantation model. This figure shows the effects of the treatment with zonisamide or neurectomy on mRNA expression.
Figure 13 shows mRNA expression levels of Bdnf, Ngf, Ntf4, Ntrk1, Ntrk2, Map2, Mapt, and Gap43 in sciatic nerve 8 weeks after surgery in the mouse sciatic nerve autologous transplantation model. This figure shows the effects of the treatment with zonisamide or neurectomy on mRNA expression.
Figure 14 shows the effect of zonisamide to improve motor function evaluated using BMS score in severely crushed spinal cord model mouse.
Figure 15 shows the effect of zonisamide to improve motor function evaluated using rotarod test in severely crushed spinal cord model mouse.
Figure 16 shows a technique of isolating spinal cord for evaluating gene expression associated with neurite outgrowth at and around the segment of the damaged area in severely crushed spinal cord model mouse.
Figure 17 shows mRNA expression level of Bdnf in the segment of the damaged area or the segment distal to the damaged area of spinal cord in severely crushed spinal cord model mice 28 days after surgery.
Figure 18 shows mRNA expression level of Ngf in the segment of the damaged area or the segment distal to the damaged area of spinal cord in severely crushed spinal cord model mice 28 days after surgery.
Figure 19 shows mRNA expression level of Ntf4 in the segment of the damaged area or the segment distal to the damaged area of spinal cord in severely crushed spinal cord model mice 28 days after surgery.
Figure 20 shows mRNA expression level of Ntrk1 in the segment of the damaged area or the segment distal to the damaged area of spinal cord in severely crushed spinal cord model mice 28 days after surgery.
Figure 21 shows mRNA expression level of Ntrk2 in the segment of the damaged area or the segment distal to the damaged area of spinal cord in severely crushed spinal cord model mice 28 days after surgery.
Figure 22 shows mRNA expression level of Map2 in the segment of the damaged area or the segment distal to the damaged area of spinal cord in severely crushed spinal cord model mice 28 days after surgery.
Figure 23 shows mRNA expression level of Mapt in the segment of the damaged area or the segment distal to the damaged area of spinal cord in severely crushed spinal cord model mice 28 days after surgery.
Figure 24 shows mRNA expression level of Gap43 in the segment of the damaged area or the segment distal to the damaged area of spinal cord in severely crushed spinal cord model mice 28 days after surgery.

### Description of Embodiments

Zonisamide (chemical name: 3-sulfamoylmethyl-1,2-benzisoxazole or 1,2-benzisoxazole-3-methanesulfonamide) may be used in the form of an alkali metal salt thereof in the present invention. Alkali metal salts of zonisamide include sodium, potassium, and lithium salts. Preferably, the alkali metal salt is sodium or potassium salt. More preferably, the alkali metal salt is sodium salt.

Zonisamide or an alkali metal salt thereof obtained as a crystal may be one of its crystal polymorphs. Zonisamide in the present invention includes all of its crystal forms.

Zonisamide of the present invention may be present as a hydrate and/or solvate and therefore the hydrate and/or solvate of zonisamide or an alkali metal salt thereof are also included in the compound of the present invention.

Zonisamide or an alkali metal salt thereof as used herein may be in the form of prodrug. The prodrug is a functional derivative of the compound (zonisamide) of the present invention that is able to be easily converted to the desired compound (zonisamide) in a living body.

"Treatment" as used herein includes any treatment for a disease (for example, amelioration of symptom, alleviation of symptom, or suppression of progression of symptom). "Prevention" as used herein includes any prevention for a disease (for example, inhibition of symptom manifestation).

The terms Bdnf, Ngf, Ntf4, Ntrk1, Ntrk2, Map2, Mapt, Gap43, Chrne, Colq, and Rapsn as used herein mean the names of genes encoding BDNF, NGF, NT-4/5, TrkA, TrkB, MAP2, Tau, GAP43, AChRε, ColQ, and Rapsyn, respectively.

"Peripheral nerve" as used herein is composed of "somatic nerve" and "autonomic nerve". "Somatic nerve" as used herein is composed of "sensory nerve" and "motor nerve". "Autonomic nerve" as used herein is composed of "sympathetic nerve" and "parasympathetic nerve".

"Peripheral neuropathy" as used herein is a condition in which normal conduction of peripheral nerve is affected. "Peripheral neuropathy" includes motor neuropathy, sensory neuropathy, and autonomic neuropathy.

Zonisamide or an alkali metal salt thereof in the present invention treats and/or prevents "peripheral neuropathy" by regenerating neuronal cells and thus treats and/or prevents "sensory neuropathy", "motor neuropathy", and "autonomic neuropathy".

"Sensory neuropathy" includes, for example, pain, paresthesia, burning sensation, and abnormal vibratory sensation.

"Motor neuropathy" includes, for example, paralysis, numbness, muscle weakness, hyposthenia, muscle atrophy, disorder of skilled movement, wrist dorsiflexion, ankle dorsiflexion, drop foot, hyporeflexia, and vasomotor symptom.

"Autonomic neuropathy" includes abnormal sweating, dysuria, erectile dysfunction, diarrhea, constipation, dizziness, shivering, palpitation, susurrus aurium, nausea, headache, slight fever, hyperpnea, malaise, insomnia, menstruation disturbance, taste disorder, and abnormal heart rate variability.

Preferably, "peripheral neuropathy" is motor neuropathy or sensory neuropathy. More preferably, "peripheral neuropathy" is motor neuropathy.

"Peripheral neuropathy" as used herein includes various peripheral neuropathies due to different causes. The causes of peripheral neuropathy include trauma, injury due to a physical factor such as deformation, compression, blood circulation disorder, genetic factors, abnormal metabolism, intoxication, immune-response, infectious disease, cancer, kidney failure, and liver disease. Preferably, "peripheral neuropathy" is caused by trauma, injury due to a physical factor, or compression. More preferably, "peripheral neuropathy" is caused by trauma or injury due to a physical factor.

"Peripheral neuropathy" as used herein includes "peripheral neuropathy associated with spondylosis" because "peripheral neuropathy" may develop when the spine is destroyed and the peripheral nerve root is damaged. "Peripheral neuropathy associated with spondylosis" is classified, based on the damaged area of the spine, into "peripheral neuropathy associated with cervical spondylosis", "peripheral neuropathy associated with thoracic spondylosis", "peripheral neuropathy associated with lumbar spondylosis", "peripheral neuropathy associated with sacral spondylosis", or "peripheral neuropathy associated with caudal spondylosis". "Peripheral neuropathy" in the present invention may be caused by injury in any area.

"Peripheral neuropathy" as used herein is clinically classified into "mononeuropathy", "multiple mononeuropathy", or "polyneuropathy".

"Mononeuropathy" mainly results from damage of a single peripheral nerve by trauma, compression, or blood circulation disorder. "Mononeuropathy" includes myeloma-associated neuropathy, mononeuropathy associated with collagen disease (for example, an auto-immune disease or systemic lupus erythematosus), mononeuropathy associated with AL amyloidosis, diabetic mononeuropathy, and mononeuropathy associated with trauma. Preferably, "mononeuropathy" is mononeuropathy associated with collagen disease, diabetic mononeuropathy, or mononeuropathy associated with trauma. More preferably, "mononeuropathy" is diabetic mononeuropathy or mononeuropathy associated with trauma. Even more preferably, "mononeuropathy" is mononeuropathy associated with trauma.

"Multiple mononeuropathy" mainly results from a plurality of mononeuropathies caused by trauma, compression, or blood circulation disorder. Generally, multiple mononeuropathy shows left-right asymmetric and random distribution. "Multiple mononeuropathy" includes multiple mononeuropathy associated with systemic vasculitis, multiple mononeuropathy associated with systemic granulomatous lesions (such as sarcoidosis), and diabetic multiple mononeuropathy. Preferably, "multiple mononeuropathy" is diabetic multiple mononeuropathy.

"Polyneuropathy" mainly results from diffuse left-right symmetric mononeuropathy caused by genetic factors, abnormal metabolism, intoxication, or immune-response. In polyneuropathy, severe symptoms are basically developed in the distal portions of the extremities. "Polyneuropathy" includes diabetic polyneuropathy, Guillain-Barre syndrome, chronic inflammatory demyelinating polyradiculoneuropathy, Charcot-Marie-Tooth disease, and diphtheritic neuritis. Preferably, "polyneuropathy" is Charcot-Marie-Tooth disease or diabetic polyneuropathy. More preferably, "polyneuropathy" is Charcot-Marie-Tooth disease.

"Peripheral neuropathy" as used herein is pathologically classified into "axonopathy" or "myelinopathy".

"Axonopathy" is peripheral neuropathy in which axons are mainly damaged and includes, for example, peripheral neuropathy caused by Wallerian degeneration and peripheral neuropathy caused by retrograde degeneration.

"Myelinopathy" is peripheral neuropathy in which myelin sheaths degenerate and drop off, and includes, for example, peripheral neuropathy caused by primary segmental demyelination and peripheral neuropathy caused by secondary segmental demyelination.

Specific examples of "peripheral neuropathy" include, for example, "hereditary neuropathy", "acquired neuropathy," "malignancy-associated neuropathy", "toxic neuropathy", "nutrient-deficiency neuropathy", "neuropathy with unknown cause", "neuromuscular junction disorder", and "compression neuropathy".

Preferably, "peripheral neuropathy" is "acquired neuropathy", "malignancy-associated neuropathy", "toxic neuropathy", "nutrient-deficiency neuropathy", "neuropathy with unknown cause", "neuromuscular junction disorder", or "compression neuropathy". More preferably, "peripheral neuropathy" is "acquired neuropathy", "malignancy-associated neuropathy", "neuropathy with unknown cause", "neuromuscular junction disorder", or "compression neuropathy". Even more preferably, "peripheral neuropathy" is "acquired neuropathy", "neuropathy with unknown cause", "neuromuscular junction disorder", or "compression neuropathy". Most preferably, "peripheral neuropathy" is "compression neuropathy".

"Hereditary neuropathy" includes, for example, Charcot-Marie-Tooth disease, hereditary neuropathy with liability to pressure palsy, hereditary neuralgic amyotrophy, metachromatic leukodystrophy (MLD), Krabbe disease, Fabry disease, adrenoleukodystrophy (ALD), Refsum disease, Tangier disease, acute porphyria, and familial amyloid polyneuropathy. Preferably, "hereditary neuropathy" is Charcot-Marie-Tooth disease.

"Acquired neuropathy" includes inflammatory neuropathy, neuropathy associated with AL amyloidosis, diabetic neuropathy, hypothyroidism, neuropathy associated with collagen disease (such as an auto-immune disease and systemic lupus erythematosus), uremic neuropathy, neuropathy associated with chronic liver disease, Crow-Fukase syndrome, Fisher syndrome, critical illness polyneuropathy, neuropathy associated with human immunodeficiency virus, and neuropathy associated with varicella-zoster virus.

Inflammatory neuropathy includes Guillain-Barre syndrome and chronic inflammatory demyelinating polyradiculoneuropathy (CIDP).

Preferably, "acquired neuropathy" is inflammatory neuropathy or diabetic neuropathy. More preferably, "acquired neuropathy" is inflammatory neuropathy. Most preferably, "acquired neuropathy" is Guillain-Barre syndrome or chronic inflammatory demyelinating polyradiculoneuropathy (CIDP).

"Malignancy-associated neuropathy" includes paraneoplastic nerve syndrome.

"Toxic neuropathy" includes drug-induced toxic neuropathy, organic material-induced neuropathy, and metal-induced neuropathy.

Drug-induced toxic neuropathy may be caused by isoniazid, vincristine, vinblastine, cisplatin, bortezomib, chloramphenicol, metronidazole, nitrofurantoin, amiodarone, thalidomide, pyridoxine, phenytoin, disulfiram, chloroquine, or pyridoxine (vitamin B6).

Organic material-induced neuropathy includes neuropathy caused by n-hexane, alcohol, or styrene intoxication.

Metal-induced neuropathy includes neuropathy caused by arsenic, mercury, or thallium.

"Neuropathy with unknown cause" includes sensory neuropathy with unknown cause and sensory and motor polyneuropathy with unknown cause. Specifically, "Neuropathy with unknown cause" includes facial paralysis.

"Neuromuscular junction disorder" includes congenital myasthenic syndrome, myasthenia gravis, sarcopenia, botulism, Eaton Lambert syndrome, and drug-induced neuromuscular junction disorder.

Drug-induced neuromuscular junction disorder may be caused by antibiotics, insecticidal agents, curare, or nerve gases.

"Compression neuropathy" includes carpal tunnel syndrome, cubital tunnel syndrome, Guyon tunnel syndrome, musculospiral paralysis, meralgia paresthetica, common fibular nerve paralysis, thoracic outlet syndrome, and tarsal tunnel syndrome. Preferably, "compression neuropathy" is carpal tunnel syndrome, thoracic outlet syndrome, or tarsal tunnel syndrome.

More preferably, "compression neuropathy" is carpal tunnel syndrome or tarsal tunnel syndrome. Most preferably, "compression neuropathy" is carpal tunnel syndrome.

Compression neuropathy may be caused by trauma, compression (which may be associated with microtrauma), and/or repetitive stress.

Other preferable specific examples of "peripheral neuropathy" include Charcot-Marie-Tooth disease, hereditary neuropathy with liability to pressure palsy, hereditary neuralgic amyotrophy, metachromatic leukodystrophy (MLD), Krabbe disease, Fabry disease, adrenoleukodystrophy (ALD), Refsum disease, Tangier disease, acute porphyria, familial amyloid polyneuropathy, Guillain-Barre syndrome, chronic inflammatory demyelinating polyradiculoneuropathy (CIDP), neuropathy associated with AL amyloidosis, diabetic neuropathy, hypothyroidism, neuropathy associated with collagen disease (such as an auto-immune disease and systemic lupus erythematosus), uremic neuropathy, neuropathy associated with chronic liver disease, Crow-Fukase syndrome, Fisher syndrome, critical illness polyneuropathy, neuropathy associated with human immunodeficiency virus, neuropathy associated with varicella-zoster virus, facial paralysis, congenital myasthenic syndrome, myasthenia gravis, sarcopenia, botulism, Eaton Lambert syndrome, drug-induced neuromuscular junction disorder, carpal tunnel syndrome, cubital tunnel syndrome, Guyon tunnel syndrome, musculospiral paralysis, meralgia paresthetica, common fibular nerve paralysis, thoracic outlet syndrome, and tarsal tunnel syndrome.

Preferably, "peripheral neuropathy" is Charcot-Marie-Tooth disease, Guillain-Barre syndrome, chronic inflammatory demyelinating polyradiculoneuropathy (CIDP), diabetic neuropathy, facial paralysis, congenital myasthenic syndrome, myasthenia gravis, sarcopenia, Eaton Lambert syndrome, drug-induced neuromuscular junction disorder, carpal tunnel syndrome, cubital tunnel syndrome, musculospiral paralysis, meralgia paresthetica, common fibular nerve paralysis, thoracic outlet syndrome, or tarsal tunnel syndrome.

More preferably, "peripheral neuropathy" is Charcot-Marie-Tooth disease, Guillain-Barre syndrome, chronic inflammatory demyelinating polyradiculoneuropathy (CIDP), diabetic neuropathy, facial paralysis, congenital myasthenic syndrome, myasthenia gravis, sarcopenia, Eaton Lambert syndrome, carpal tunnel syndrome, cubital tunnel syndrome, thoracic outlet syndrome, or tarsal tunnel syndrome.

Even more preferably, "peripheral neuropathy" is Charcot-Marie-Tooth disease, Guillain-Barre syndrome, chronic inflammatory demyelinating polyradiculoneuropathy (CIDP), diabetic neuropathy, facial paralysis, myasthenia gravis, or carpal tunnel syndrome.

Most preferably, "peripheral neuropathy" is Charcot-Marie-Tooth disease, chronic inflammatory demyelinating polyradiculoneuropathy (CIDP), or carpal tunnel syndrome.

"Spine" as used herein is composed of "cervical vertebrae", "thoracic vertebrae", "lumbar vertebrae", "sacral vertebrae", and "caudal vertebrae".

"Spinal cord" as used herein is composed of "cervical spinal cord", "thoracic spinal cord", "lumbar spinal cord", "sacral spinal cord", and "coccygeal spinal cord".

"Spinal cord injury" as used herein is a condition in which the spinal cord is damaged mainly because the spine is destroyed due to the strong external force applied to vertebral column. "Spinal cord injury" includes spinal cord injury associated with "motor dysfunction", spinal cord injury associated with "sensory dysfunction", and spinal cord injury associated with "autonomic dysfunction".

Zonisamide or an alkali metal salt thereof in the present invention treats and/or prevents "spinal cord injury" and thus treats and/or prevents "motor dysfunction", "sensory dysfunction", and "autonomic dysfunction" associated with spinal cord injury.

"Motor dysfunction" includes paralysis, numbness, muscle weakness, hyposthenia, disorder of skilled movement, wrist dorsiflexion, ankle dorsiflexion, drop foot, hyporeflexia, vasomotor symptom, convulsion, spasticity, and contracture.

Preferably, "motor dysfunction" is paralysis, numbness, muscle weakness, hyposthenia, hyporeflexia, convulsion, spasticity, or contracture. More preferably, "motor dysfunction" is paralysis, numbness, muscle weakness, hyporeflexia, or convulsion. Even more preferably, "motor dysfunction" is paralysis, numbness, muscle weakness, or hyporeflexia.

Most preferably, "motor dysfunction" is paralysis and numbness.

"Sensory neuropathy" includes pain, paresthesia, and phantom limb pain.

"Autonomic dysfunction" includes abnormal sweating, impaired excretion, erectile dysfunction, diarrhea, constipation, dizziness, shivering, palpitation, susurrus aurium, nausea, headache, slight fever, hyperpnea, malaise, insomnia, menstruation disturbance, taste disorder, and abnormal heart rate variability.

Preferably, "spinal cord injury" includes spinal cord injury associated with "motor dysfunction" and spinal cord injury associated with "sensory dysfunction". More preferably, "spinal cord injury" is spinal cord injury associated with "motor dysfunction". Preferably, dysfunctions associated with "spinal cord injury" is "motor dysfunction" or "sensory dysfunction". More preferably, dysfunctions associated with "spinal cord injury" is "motor dysfunction".

"Spinal cord injury" as used herein is classified into "cervical spinal cord injury", "thoracic spinal cord injury", "lumbar spinal cord injury", "sacral spinal cord injury", or "coccygeal spinal cord injury" based on the damaged area. "Spinal cord injury" in the present invention may be injury in any area.

"Spinal cord injury" as used herein is classified into "complete" or "incomplete" spinal cord injury based on the severity of spinal cord injury. In "complete" spinal cord injury the spinal cord is transected and completely lacks neurotransmitting function, whereas in "incomplete" spinal cord injury the spinal cord is partially broken or compressed and keeps some functions. "Spinal cord injury" in the present invention includes both "complete" and "incomplete" spinal cord injuries.

"Spinal cord injury" as used herein includes "spinal cord injury" caused by any of different causes. The causes of "spinal cord injury" include trauma, injury due to a physical factor such as deformation, compression, blood circulation disorder, or infectious disease. Preferably, "spinal cord injury" is caused by trauma, injury due to a physical factor, or compression. More preferably, "spinal cord injury" is caused by trauma or injury due to a physical factor.

"Spinal cord injury" as used herein includes "spinal cord injury associated with spondylosis". "Spinal cord injury associated with spondylosis" is classified into, based on the damaged area of the spine, "spinal cord injury associated with cervical spondylosis", "spinal cord injury associated with thoracic spondylosis", "spinal cord injury associated with lumbar spondylosis", "spinal cord injury associated with sacral spondylosis", or "spinal injury associated with caudal spondylosis". "Spinal cord injury" in the present invention may be associated with injury in any area.

The active ingredient of the present invention (zonisamide or an alkali metal salt thereof) may be formulated into a suitable dosage form and administered by an oral or parenteral route. The dosage form includes, but is not limited to, for example, a tablet, capsule, powder, granule, liquid, suspension, injection, patch, and cataplasm. The formulation is produced using a pharmaceutically acceptable additive by any known method.

The additive may be, depending on the purpose, an excipient, disintegrating agent, bonding agent, fluidizing agent, lubricating agent, coating agent, solubilizing agent, dissolution aid, thickening agent, dispersing agent, stabilizing agent, sweetening agent, or flavoring agent. Specifically, the additive may be lactose, mannitol, crystalline cellulose, low-substituted hydroxypropyl cellulose, corn starch, partially pregelatinized starch, light anhydrous silicic acid, croscarmellose calcium, croscarmellose sodium, hydroxypropyl cellulose, hydroxypropyl methylcellulose, polyvinyl alcohol, ethyl cellulose, magnesium stearate, sodium stearyl fumarate, polyetheylene glycol, propylene glycol, aspartame, titanium oxide, or talc.

The compound of the present invention may be administered by any of oral, parenteral, and rectal routes. The daily dosage may vary depending on the factors such as the type of the compound, route of administration, and symptom and age of the patient. For example, the daily oral dosage that is expected to be effective for adults is from 0.1 mg to 3000 mg, 1 mg to 2000 mg, 10 mg to 1000 mg, or 20 mg to 600 mg per day in a single dose or divided doses, depending on the symptom.

For example, the daily intravenous dosage that is expected to be effective for adults is 0.01 mg to 1000 mg, 0.1 mg to 500 mg, 1 mg to 200 mg, or 1 mg to 50 mg per day in a single dose or divided doses, depending on the symptom.

When zonisamide or an alkali metal salt thereof, which is an active compound of the present invention, is formulated (into a pharmaceutical formulation or pharmaceutical composition) with or without another drug, the active ingredient (s) may be comprised in total, for example, without limitation, 0.1 to 70% by weight relative to the total constituents in the formulation. Preferably, the active ingredient(s) is comprised in total 5 to 60% by weight relative to the total constituents in the formulation. More preferably, the active ingredient(s) is comprised in total 10 to 55% by weight relative to the total constituents in the formulation.

### Examples

The present invention will be described in detail in Examples below but is not limited thereto.

### Example 1

### Screening in NSC34 cells and primary spinal cord motor neurons

### Method of screening in NSC34 cells

NSC34 cells (mouse neuroblastoma-spinal cord hybrid cells displaying a multipolar motor neuron-like phenotype; a cell line in which GFP was stably induced by doxycycline (NSC34-pTetR12-TO/GFP), Dev Dyn 1992, 194, 209-221) were cultured in Dulbecco's Modified Eagle's Medium (DMEM, Invitrogen) supplemented with 10% fetal bovine serum (FBS, Thermo Scientific) in a humidity of 95% under an atmosphere of 5% carbon dioxide at 37°C. The cells were seeded in DMEM/F12 differentiation medium (Invitrogen) supplemented with 1% FBS and 2 mg/mL doxycycline in a 96-well plate at 1.6 × 10³ cells/cm² to induce the expression of GFP. At day 1, the differentiation medium was exchanged to DMEM/F12 medium supplemented with 1% NEAA (Non-Essential Amino Acid, MP Biomedicals) and 2 mg/mL doxycycline and the cells were incubated in the presence of 10 µmol/L of each of test compounds (1186 FDA-approved drugs) for 48 hours. Subsequently, cell nuclei were stained with Hoechst 33342 (1:100,000, Sigma Aldrich). Sixteen images per well were acquired on ArrayScan VTI HCS Reader (Thermo Scientific Cellomics). The average of neurite length in each well was automatically determined using Neuronal Profiling v4.0 BioApplication (Thermo Scientific Cellomics).

### Method of screening in primary spinal cord motor neurons

Primary cultured mouse embryonic spinal cord motor neurons were isolated from spinal cord embryo of C57BL/6J mouse on 13.5th day of embryonic stage (E13.5). Solutions of zonisamide in DMSO were prepared at concentrations from 200 µmol/L to 2 mmol/L and then added to the culture medium to obtain the final concentration of zonisamide from 1 µmol/L to 20 µmol/L. The culture medium contained 0.5% DMSO whether or not it contained zonisamide. The cells were seeded at 4.0 × 10³ cells/cm² in a 96-well plate (Asahi Techno Glass) coated with poly-L-lysine and laminin and maintained in Sumilon neuron culture medium (Sumitomo Bakelite). After incubation for 2 days, the cells were fixed in PBS with 4% formamide at ordinary temperature for 15 minutes. The cells were washed with PBS once, blocked with PBS containing 2% goat serum and 0.1% Triton-X, and immunostained with mouse anti-tau-1 monoclonal antibody (1:500, MAB3420, Millipore) and Alexa Fluor 555-conjugated goat anti-mouse secondary antibody (1:1000, Life Technologies). Neurites were automatically analyzed on ArrayScan VTI HCS Reader. The neurite length and the number of nerve branch points were quantified by evaluating the longer 200 neurites. The ratio of neurite-bearing cells was determined by counting cells having a neurite length of 25 micron or more in each well.

### Results of screening in NSC34 cells

Amoung 1186 test compounds, about 100 compounds increased the average neurite length of NSC34 cells. From the 100 compounds, 20 compounds were eliminated because of inability of systemic administration or their toxicity. The rest 80 compounds were screened by repeating a similar assay 16 times, and 16 drugs were selected.

### Results of screening in primary spinal cord motor neurons

The 16 drugs were added to primary motor neurons isolated from spinal cord of C57/BL6J mouse at E13.5. After culture for 48 hours, axons were immunofluorescently stained with tau and neurite lengths were then automatically measured on ArrayScan VTI HCS Reader. Consequently, it was found that zonisamide was able to extend neurites of primary motor neurons.

### Results of evaluation of dose dependency

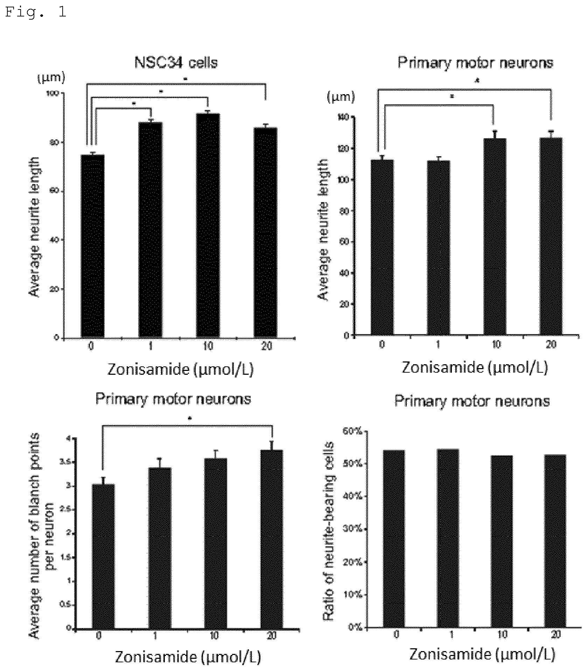

Zonisamide was examined for its neurite outgrowth effect on spinal cord motor neurons in a dose-dependent manner (Figure 1). Zonisamide induced a dose-dependent axonal growth in NSC34 cells and primary motor neurons. Zonisamide further showed a dose-dependent increase in the number of branch points in axons of primary motor neurons. However, the ratio of neurite-bearing cells in primary motor neurons was not increased by zonisamide administration.

### Example 2

### Evaluation of neurite regeneration effect by using scratch assay

### Method of scratch assay

Primary cultured motor neurons were seeded in a Nun Lab-Tek Chamber Slide (Thermo Scientific) coated with poly-L-lysine at 8.0 × 10⁴ cells/cm² × 0.7 cm²/well and then cultured in Sumilon neuron culture medium for 48 hours. Neurites of primary motor neurons were linearly scratched off with a 200 µL sterile pipette tip. The cells were immediately rinsed with PBS and cultured in Sumilon neuron culture medium containing 0, 1, 10, or 20 µmol/L zonisamide for 48 hours. The cells were fixed in 4% paraformaldehyde and immunostained with neuron-specific β-III tubulin monoclonal antibody (1:1000, MAB1195, R&D systems) and Alexa Fluor 488-conjugated goat anti-mouse secondary antibody (Life Technologies). Images of the scratched areas were acquired on a microscope (Olympus LX71). The neurite lengths of primary motor neurons in the scratched areas were automatically measured with MetaMorph software (Universal Imaging).

### Results of scratch assay

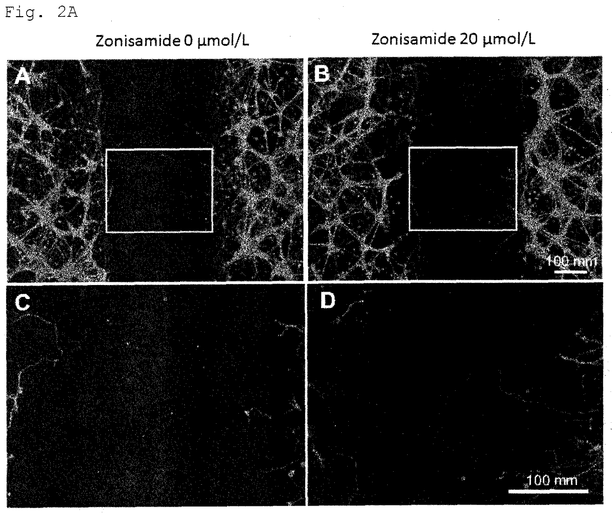

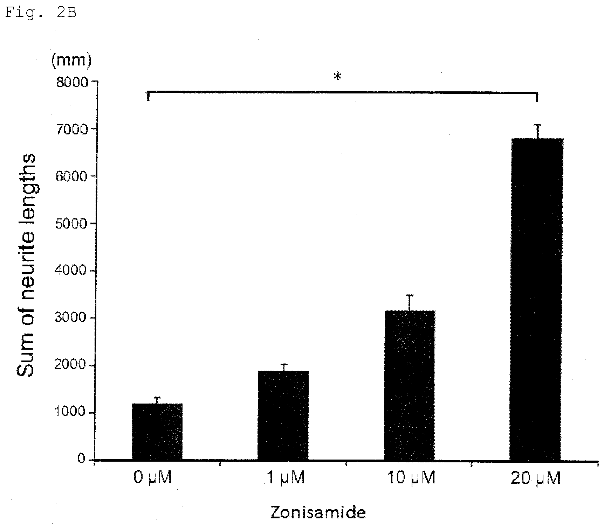

Zonisamide induced a dose-dependent increase in neurite length in the scratch assay (Figures 2A and 2B). Zonisamide was shown to promote neurite regeneration of primary motor neurons.

### Example 3

### Time-lapse imaging of axonal growth of primary motor neurons

### Method of imaging

Primary cultured motor neurons were seeded in a 96-well plate at 6.0 × 10³ cells/well in Sumilon neuron culture medium to induce neurite outgrowth. Phase-contrast images were taken every 8 hours for 3 days on IncuCyte ZOOM Live Cell Imaging System (Essen Bioscience). The neurite length was automatically analyzed with NeuroTrack software (IncuCyte).

### Results

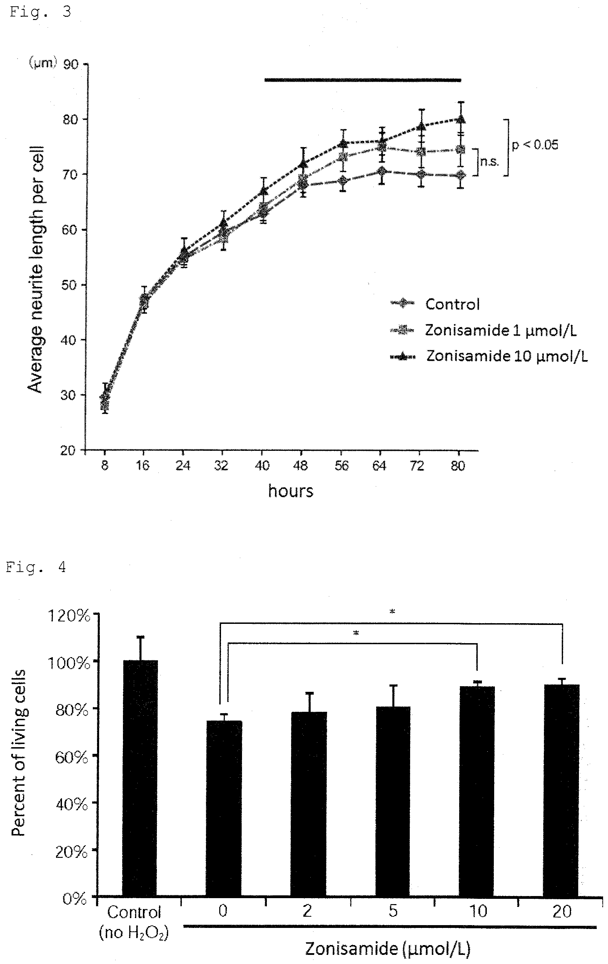

Forty hours after zonisamide administration, neurite outgrowth effect was observed in mouse primary motor neurons (Figure 3). The outgrowth effect was higher in the 10 µmol/L zonisamide administration group than in the 1 µmol/L zonisamide administration group throughout the observation period. Zonisamide was shown to enhance neurite outgrowth rather than to initiate neurite outgrowth.

### Example 4

### Evaluation of effects on oxidative stress by using MTS assay

### Method of MTS assay

Cell survival rate was assessed by MTS assay (CellTiter 96 Aqueous One Solution Cell Proliferation Assay, Promega), which measured the activity of mitochondrial reductase. Primary cultured motor neurons were cultured in Sumilon neuron culture medium for 24 hours, and the medium was then exchanged to DMEM/F12 supplemented with 0.5% FBS and cultured for 24 hours. Zonisamide was added at various concentrations for 1 hour and 100 µmol/L hydrogen peroxide was then added for 24 hours. The cells were incubated with MTS reagent for 2 hours. MTS signal was quantified on a microplate reader (PowerScan HT, DS PharmaBiomedical). The group of cells treated without hydrogen peroxide was used as a control for evaluating MTS signal.

### Results of MTS assay

Zonisamide significantly increased the number of living cells in primary motor neurons in a dose-dependent manner (Figure 4). Zonisamide was shown to have neuroprotective effect on cell death caused by oxidative stress in primary motor neurons.

### Example 5

### Evaluation of gene expression associated with neurite outgrowth

### Methods of total RNA extraction and real-time RT-PCR analysis

Total RNA was isolated, using Trizol (Thermo Fisher Scientific), from primary motor neurons cultured with 0, 1, or 10 µmol/L zonisamide. Similarly, total RNA was isolated from sciatic nerve segments with a length of 3 mm or from tibialis anterior muscle. Single-stranded cDNA was synthesized using ReverTra Ace (Toyobo). The mRNA expression levels of Bdnf, Ngf, Ntf4, Ntrk1, Ntrk2, Map2, Mapt, and Gap43 were quantified using LightCycler 480 Real-Time PCR (Roche) and SYBR Green (Takara). The mRNA levels were normalized with the expression level of Gapdh.

### Results

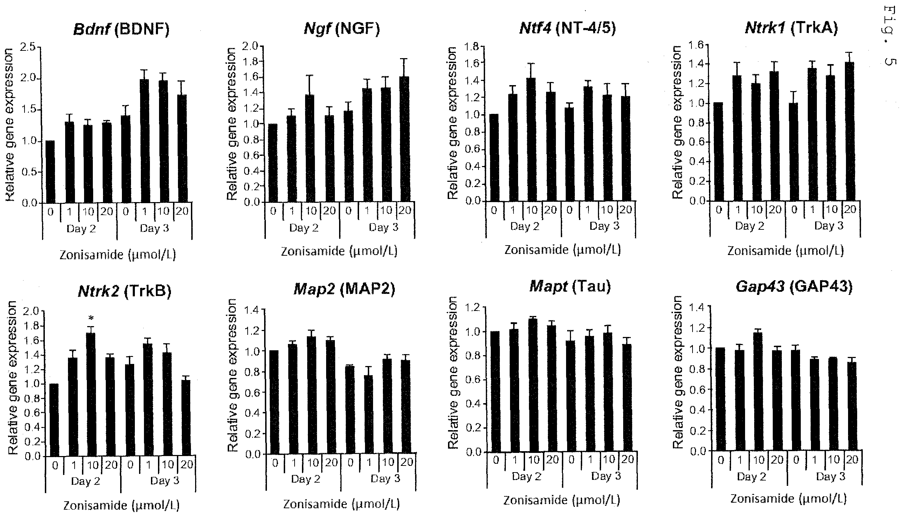

It was shown that the mRNA levels of BDNF, NGF, and NTF4 (neurotrophins) were increased 2 and 3 days after zonisamide addition (Figure 5). The mRNA levels of their receptors, NTRK1 (receptor for NGF) and NTRK2 (receptor for BDNF and NT4-4/5) were also increased by zonisamide administration. The mRNA levels of structural proteins, MAP2 (abundant in dendrites), MAPT (abundant in axon), and GAP43 (abundant in growth cone), were not increased until day 3. Zonisamide elevated the expression levels of neurotrophins and their receptors.

### Example 6

### Determination of phosphorylation level of ERK1/2 and JNK1/2/3

### Method of Western blot

Zonisamide (0, 10, or 20 µmol/L) was added to primary motor neurons 4 hours after the beginning of culture. The cells were rinsed with a buffer (50 mmol/L HEPES pH 7.0, 150 mmol/L NaCl, 1% glycerol, 1% TritonX-100, 1.5 mmol/L MgCl₂, 1 mmol/L EGTA, 100 mmol/L NaF, 10 mmol/L sodium pyrophosphate, 1 mg/mL aprotinin, 1 mg/mL leupeptin, 1 mg/mL pepstatin A, 1 mmol/L PMSF, and 1 mmol/L sodium orthovanadate (V)) at days 2 and 3. Total protein was dissolved in 1 × Laemmli buffer, separated on a 10% SDS polyacrylamide gel, and transferred to a polyvinylidene fluoride membrane (Immobilon-P, Millipore). The membrane was washed with Tris-buffered physiological saline with 0.05% Tween 20 (TBS-T) and blocked with TBS-T containing 3% skim milk at room temperature for 1 hour. The membrane was incubated with any of anti-Erk1/2 (#4696, Cell Signaling), anti-phosphorylated Erk1/2 (#4370, Cell Signaling), or anti-phosphorylated JNK1/2/3 (#4668, Cell Signaling) antibody (diluted at 1:1000) at 4°C overnight. The membrane was washed with TBS-T for 10 minutes three times and incubated with mouse anti-mouse IgG secondary antibody conjugated to horseradish peroxidase (HRP, GE Healthcare, 1:6000) at room temperature for 1 hour. The blot was detected with Amersham ECL Western blotting detection reagent (GE Healthcare) and quantified with ImageJ program (http://imagej.nih.gov/ij/index.html).

### Results

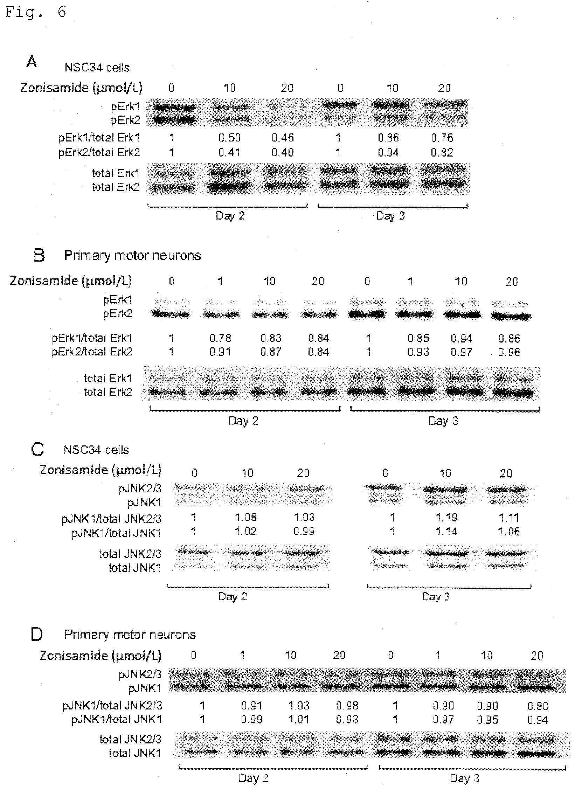

Zonisamide was shown to suppress Erk1/2 phosphorylation when the phosphorylation levels of Erk1/2 and JNK1/2/3 were measured in primary motor neurons (Figure 6). In contrast, zonisamide slightly increased the phosphorylation level of JNK1/2/3 in NSC34 cells and did not affect that level in primary motor neurons.

### Example 7

### Evaluation in mouse sciatic nerve autologous transplantation model

### Method of evaluation

Adult male C57BL/6J mice (8 weeks old; 19.5-22.0 g) were purchased from Charles River. The mice were anesthetized with isoflurane and underwent gluteal muscle incision under a sterile condition to expose the sciatic nerve in the left leg. The sciatic nerve was cut at the following two sites (1) and (2):
(1) the site 3 mm distal to the first bifurcation of biceps femoris (hereinafter referred to as proximal transection site)
(2) the site 3 mm distal to the proximal transection site (hereinafter referred to as distal transection site)

The excised nerve segment with a length of 3 mm was confirmed to be completely cut at the two sites. The perineurium was sutured with nylon thread (10-0 black) at the proximal transection site and the distal transection site under a microscope. The remaining thread was used as a marker for finding the nerve transection site in the sacrifice of the mice. In order to reduce DMSO toxicity, a solution of 60 mg/mL zonisamide in 100% DMSO was prepared and the solution was diluted in olive oil to give a solution of 3 mg/mL zonisamide in 5% DMSO. The control solution (5% DMSO) or zonisamide (3 mg/mL × about 0.2 mL) at 30 mg/kg/day was administered to the stomach using a disposable probe needle (flexible type, Fuchigami) once a day for 1 to 8 weeks from the end of surgery until sacrifice. Myelin sheath formation in the sciatic nerve was analyzed in six mice in each group. One week after the sciatic nerve autologous transplantation, the sciatic nerve was transected at "the proximal transection site" and "the site 3 mm distal to the distal transection site" to excise a nerve segment with a length of 6 mm. Subsequently, the nerve segment was fixed in 2% paraformaldehyde/2.5% glutaraldehyde in 0.2 mol/L sodium cacodylate buffer pH 7.3 overnight, treated with 2% osmium tetroxide for 3 hours, and dehydrated in ethanol. After epon embedding, a semithin section (with a thickness of 1 µm) was cut at the position 0.7 mm distal to the distal transection site, stained with alkaline Toluidine blue (Sigma), and examined under a microscope (FSX100, Olympus). The number and area of myelinated axons in the sciatic nerve were manually measured using ImageJ software in a blinded fashion.

Motor function was assessed every week in three mice in each group using the walking track analysis. In the walking track analysis, footprints of both hindlimbs were recorded when mice were allowed to walk freely on a runway. Sciatic function index (SFI; Bain et al. Plast Reconstr Surg 83: 129-138 (1989)) was calculated by measuring the width and length of footprints of both legs in a blinded fashion. The SFI was set to 0 when there was no difference between the hindlimb with transected sciatic nerve and the hindlimb without transected sciatic nerve. On the other hand, the SFI was set to -100 when the hindlimb with transected sciatic nerve became completely paralyzed. Prior to recording footprints, mice were allowed to practice walking on the runway several times. Footprints were recorded on six to eight thin boards.

Eight weeks after sciatic nerve autologous transplantation, the entire gastrocnemius muscle and a sciatic nerve segment between the distal transection site and the site 3 mm distal to the distal transection site were isolated for total RNA extraction. Similar sciatic nerve segment was also isolated from the hindlimb without transected sciatic nerve. The mRNA expression levels of Bdnf, Ngf, Ntf4, Ntrk1, Ntrk2, Map2, Mapt, Gap43, Chrne, Colq, and Rapsn were quantified in a manner similar to Example 5.

Eight weeks after sciatic nerve autologous transplantation, the tibialis anterior muscle was isolated and fixed in cold 100% methanol for hematoxylin and eosin staining.

### Results

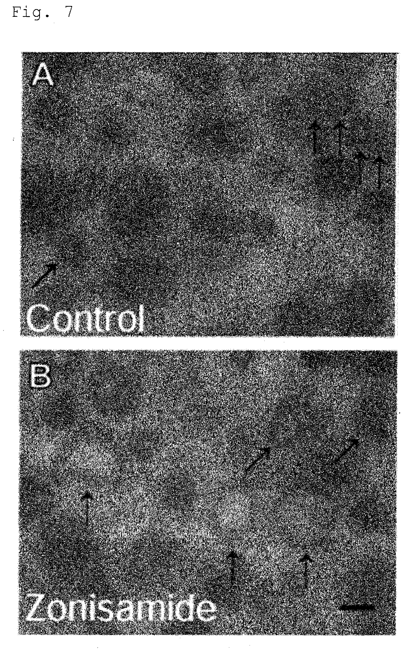

In vivo nerve regeneration effect of zonisamide was assessed in the sciatic nerve autologous transplantation model. Zonisamide was administered to the stomach at 30 mg/kg/day every day after surgery. The section at the position 0.7 mm distal to the distal transection site was observed one week after surgery. It was revealed that the number of axons was unchanged but the area of axons was increased 3.9 fold compared to the zonisamide non-administration group (Figure 7).

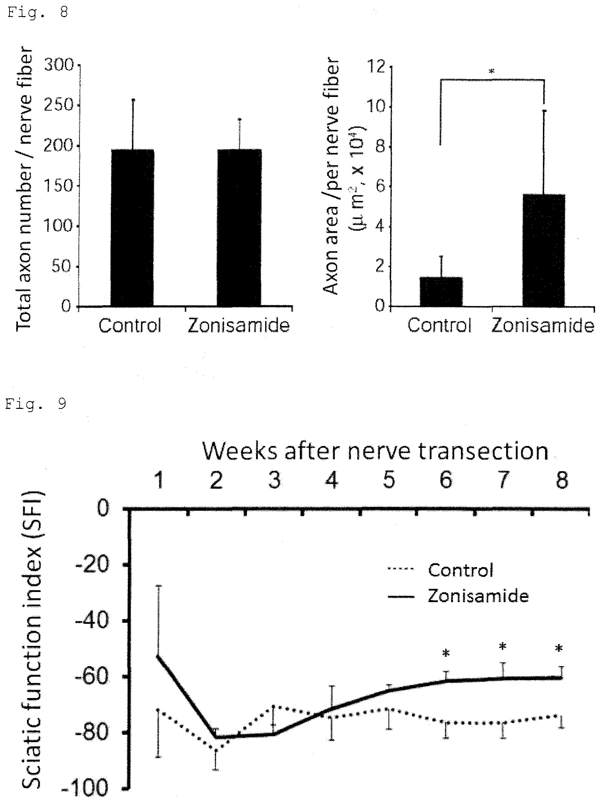

Effects of chronic administration of zonisamide on motor function and muscles (tibialis anterior muscle and gastrocnemius muscle) were examined. It was revealed that SFI, a marker of motor function of hindlimbs after sciatic nerve autologous transplantation, was dramatically improved in the group of mice given zonisamide at 6 to 8 weeks after surgery (Figure 9).

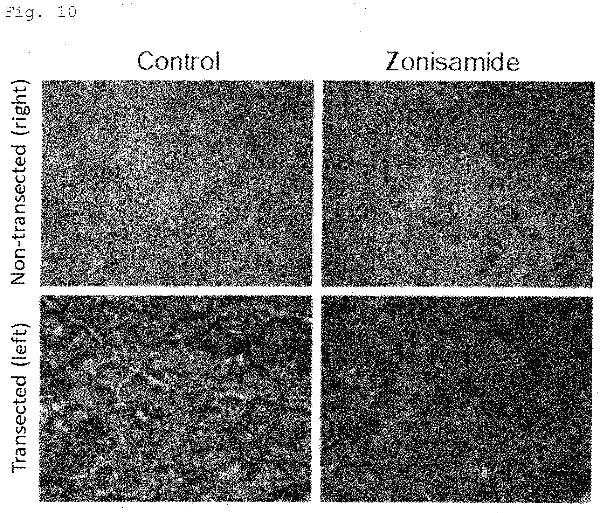

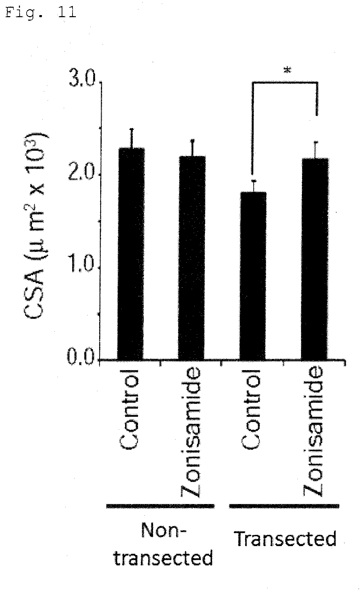

The cross sections of tibialis anterior muscle were histologically and morphologically analyzed and it was revealed that zonisamide promoted recovery from denervation atrophy (Figure 10, Figure 11).

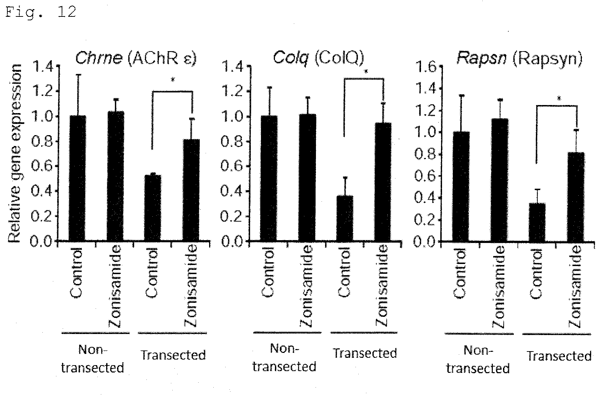

It was demonstrated that the expression levels of genes specifically expressed in the neuromuscular junction, including Chrne (Jones et al. Proc Natl Acad Sci U S A 93: 5985-5990 (1996)), Colq (Trinkaus et al. J Neurochem 105: 2535-2544 (2008)), and Rapsn (Brockhausen et al. Dev Neurobiol 68: 1153-1169 (2008)), were increased in the zonisamide administration group (Figure 12) .

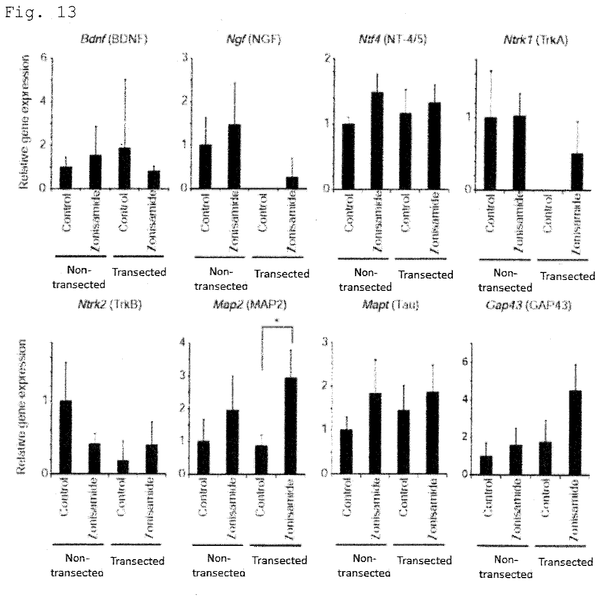

The gene expression levels were analyzed in the sciatic nerve segment distal to the distal transection site, and it was revealed that the expression level of Bdnf was unchanged but the gene expression levels of Ngf and Ntf4 were increased at 8 weeks after surgery in mice given zonisamide (Figure 13). Moreover, it was demonstrated that mRNA levels of their receptors, Ntrk1 and Ntrk2, were also increased by zonisamide. Also, mRNA levels of structural proteins, Map2, Mapt, and Gap43, were elevated in the zonisamide administration group.

These results demonstrate that zonisamide induces axon outgrowth and recovery of sciatic nerve function.

### Example 8

### Evaluation of motor function in severely crushed spinal cord model mice

### Method of producing severely crushed spinal cord model mice

Female C57BL/6J mice (8 weeks old; 19.5-22.0 g) were purchased from Charles River. The female C57BL/6J mice were anesthetized by intraperitoneally administering pentobarbital sodium and underwent back incision under a sterile condition to perform laminectomy at the 10th thoracic vertebra. The spinal cord was crushed at 100 kdyn using a tip with a diameter of 1.35 mm on IH Spinal Cord Impactor. After the occurrence of hematoma in the spinal cord was confirmed under a microscope, the wound was closed and 1 ml of physiological saline was subcutaneously injected into the posterior neck region to prevent dehydration. The mice with spinal cord injury were separated into two groups in a blinded fashion. Zonisamide was diluted with olive oil (solvent) to 3 mg/mL and intragastrically administered to mice in one group at 30 mg/kg/day using a disposable probe needle (flexible type, Fuchigami) for consecutive days from the day of surgery. Mice in the other group received only the solvent using a similar procedure for consecutive days. One day after surgery, motor function of mice in both groups was assessed using BMS score (Basso mouse scale; Li et.al. J Neurosurg Spine 4: 165-173 (2006)) in a blinded fashion to select the mice with BMS score of 1 or lower and consider them as severely crushed spinal cord model mice. The mice received 200 mL of water containing trimethoprim (16 mg) /sulfamethoxazole (80 mg) from a feeder to prevent infection for 3 days after surgery.

Thus, "the group of severely crushed spinal cord model mice that received zonisamide (zonisamide administration group)" and "the control group of severely crushed spinal cord model mice that received only the solvent (control group)" were produced.

### BMS score

BMS score is scored by rating ankle movement, weight support, stepping, coordinated motion of forelimbs and hindlimbs (coordination), hindlimb position in walking, trunk stability, and tail position, and ranges from 0 to 9; score 0 corresponds to complete paralysis of hindlimb and score 9 corresponds to normal motion; more specifically, BMS score is described as follows:
0: No ankle movement.
1: Slight ankle movement (less than 50% of the range of motion).
2: Extensive ankle movement (equal to and greater than 50% of the range of motion).
3: Plantar placing of the paw or occasional dorsal stepping.
4: Occasional planter stepping with weight support (equal to and less than 50% of forward movement).
5: Frequent or consistent planter stepping (equal to and greater than 50% of forward movement), no coordination of limbs;
or frequent or consistent planter stepping, some coordination of limbs, hindlimb paws rotated at initial contact and lift off.
6: Some coordination of limbs, hindlimb paws parallel at initial contact and rotated at lift off; or mostly coordinated, hindlimb paws rotated at initial contact and lift off.
7: Mostly coordinated, hindlimb paws parallel at initial contact; or mostly coordinated, hindlimb paws parallel at initial contact and lift off, and severe trunk instability.
8: Hindlimb paws parallel at initial contact and lift off, and mild trunk instability; or hindlimb paws parallel at initial contact and lift off, and normal trunk stability and the tail down or up and down.
9: Normal trunk stability and tail always up.

### Results

### Evaluation of motor function with BMS score

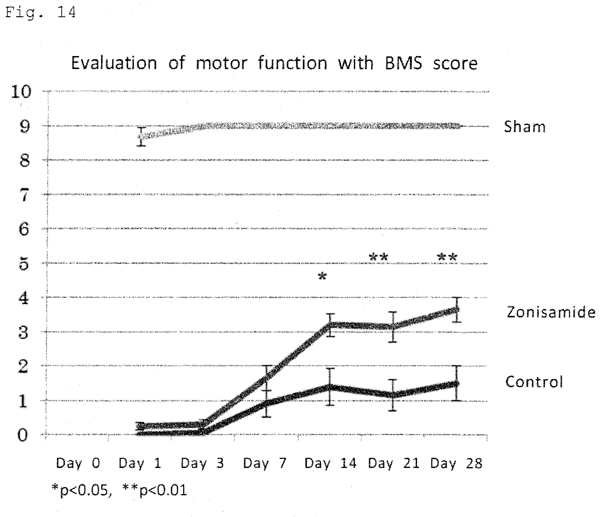

"Ten mice in the zonisamide administration group", "ten mice in the control group", and "three mice in the Sham group, which underwent only laminectomy without spinal cord crush and received only the solvent (the Sham group)" were used to assess their motor function. A solution of zonisamide diluted in the solvent was administered to "the zonisamide administration group" and the solvent was administered to "the control group" and "the Sham group" according to the procedure described in "Method of producing severely crushed spinal cord model mice" for consecutive days from the day of injury. BMS scores were measured at days 1, 3, 7, 14, 21, and 28 after surgery in a blinded fashion (Figure 14).

BMS score in the Sham group, which underwent only laminectomy, was the maximum score 9 since day 3 after surgery. BMS score in the zonisamide group was significantly higher than that in the control group since day 14 after surgery. BMS scores at day 14 after surgery in the zonisamide administration group and the control group were 3.2 ± 0.3 and 1.4 ± 0.5, respectively (p=0.014). BMS scores at day 21 after surgery in the zonisamide administration group and the control group were 3.2 ± 0.4 and 1.2 ± 0.5, respectively (p=0.008). BMS scores at day 28 after surgery in the zonisamide administration group and the control group were 3.7 ± 0.4 and 1.5 ± 0.5, respectively (p=0.005).

### Evaluation of motor function in rotarod test

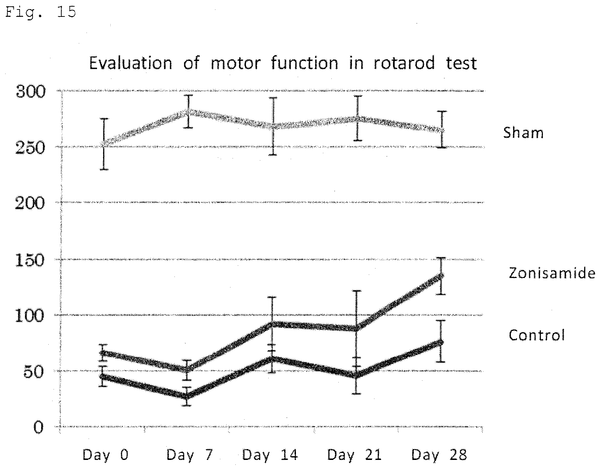

"Four mice in the zonisamide administration group", "four mice in the control group", and "three mice in the Sham group" were used to assess their motor function using a rotarod (from UGO BASILE) at the day of surgery, days 1, 7, 14, 21, and 28 after surgery by administering the drug for consecutive days from the day of injury in a fashion similar to the "Evaluation of motor function with BMS score". The latency until a mouse fell off the rotarod (holding time, in seconds; a maximum latency of 300 seconds) was measured in the accelerating rotation mode with an initial speed of 2 rpm, an accelerated speed of 5 rpm/min, and a maximum speed of 40 rpm. The latency was measured 3 times at an interval of 10 minutes and the three measurements were averaged for assessment (Figure 15) .

The holding time in the Sham group was 250 seconds or more since the day following the surgery. The holding time in the zonisamide administration group was shown to be longer than that in the control group at days 21 and 28 after surgery. At day 28 after surgery, the holding time was 135.3 ± 16.4 seconds in the zonisamide administration group while the holding time was 76.6 ± 18.9 seconds in the control group (p=0.088).

The results of evaluation of motor function with BMS score and the rotarod test revealed that the motor function was improved by zonisamide administration in the severely crushed spinal cord model mice.

### Example 9

### Evaluation of gene expression associated with neurite outgrowth at and around the segment of the damaged area in severely crushed spinal cord model mice

### Methods

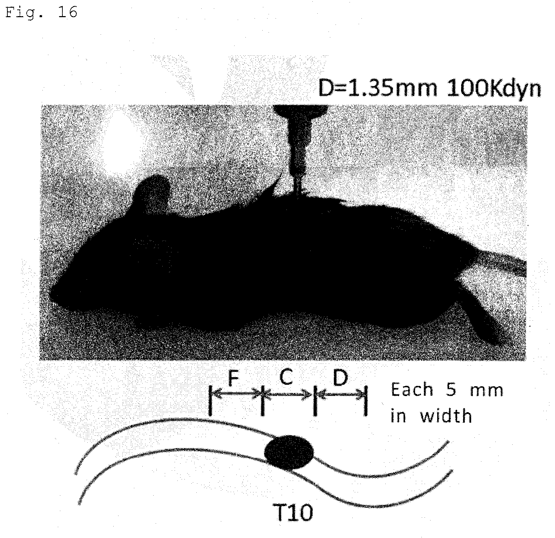

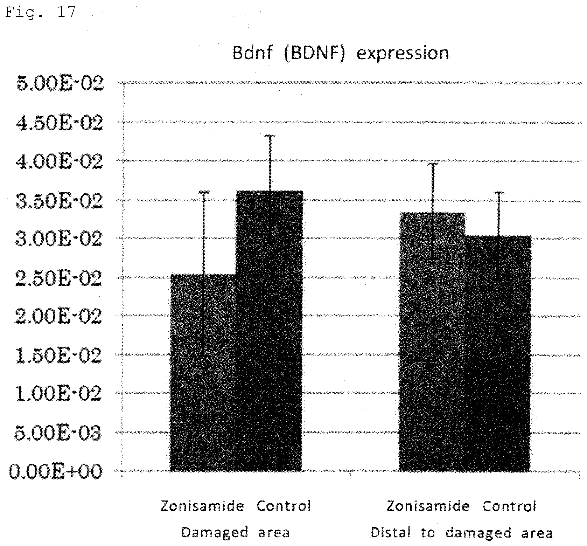

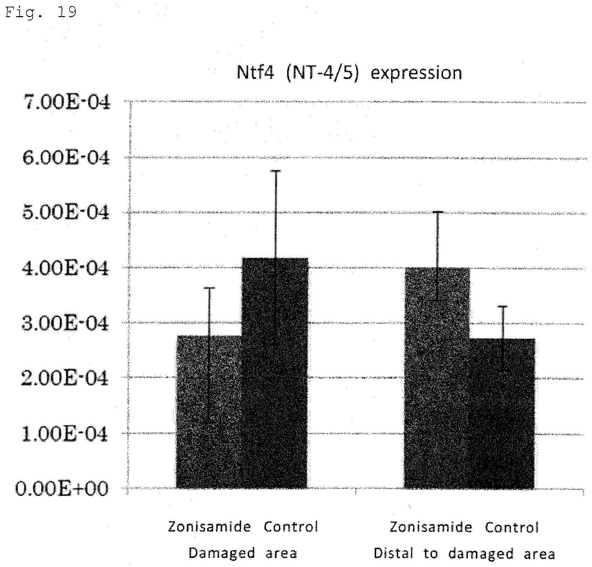

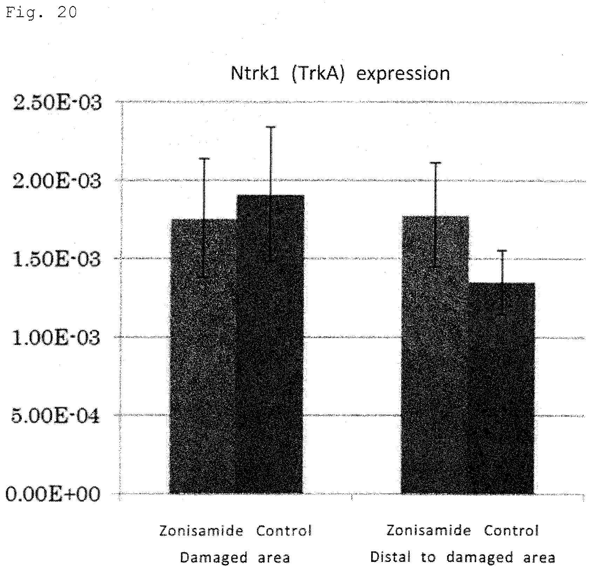

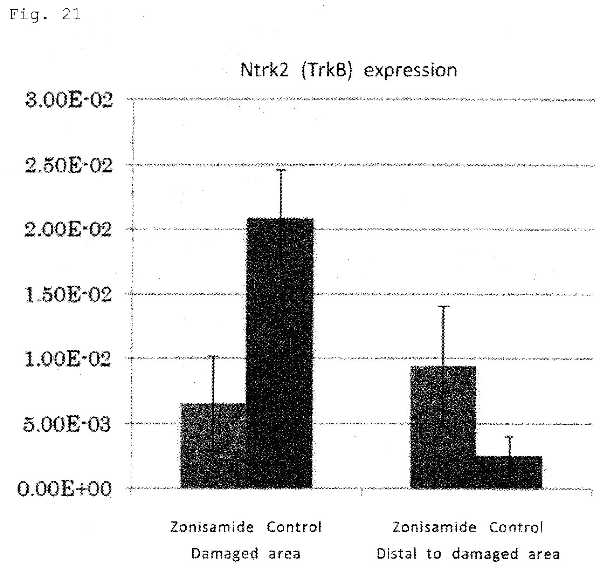

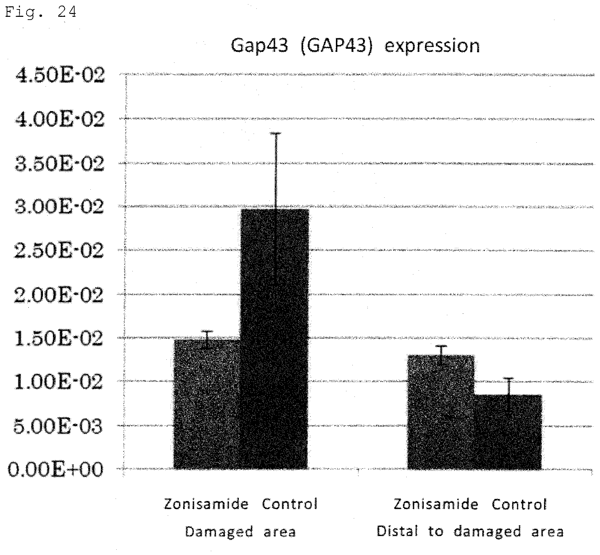

Severely crushed spinal cord model mice were produced according to the "Method of producing severely crushed spinal cord model mice" as described in Example 8. Spinal cords were isolated from four mice in the zonisamide administration group and four mice in the control group 28 days after surgery and the segment of the damaged area with a length of 5 mm and the segment with a length of 5 mm distal to the damaged area were collected (Figure 16). Total RNA was isolated using Trizol (Thermo Fisher Scientific) from each spinal cord segment with a length of 5 mm. Single-stranded cDNA was synthesized using ReverTra Ace (Toyobo). The mRNA expression levels of neurotrophic factors, Bdnf, Ngf, Ntf4, Ntrk1, Ntrk2, Map2, Mapt, and Gap43 were quantified using LightCycler 480 Real-Time PCR (Roche) and SYBR Green (Takara) (Figures 17, 18, 19, 20, 21, 22, 23, and 24). The mRNA levels were normalized with the expression level of GAPDH.

### Results

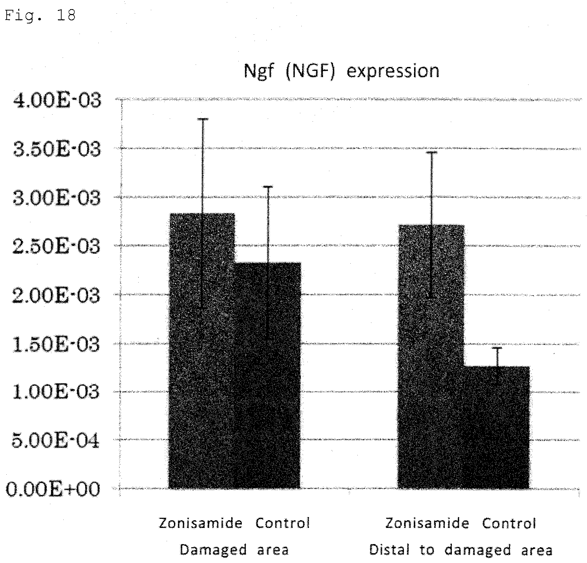

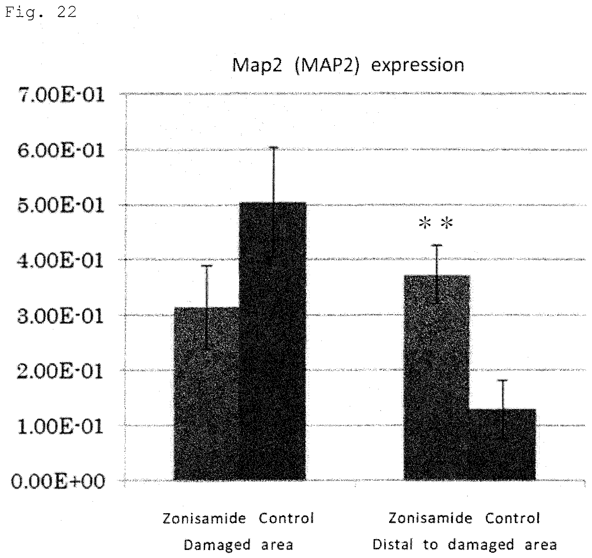

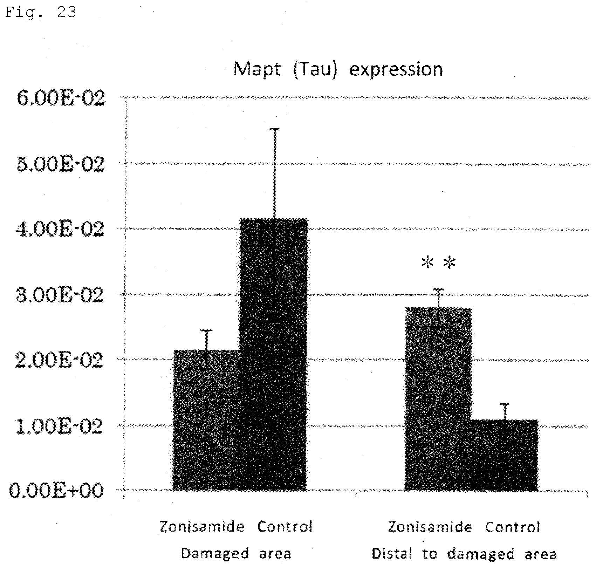

The expression levels of MAP2 and Tau genes were significantly increased in the segment distal to the damaged area of the zonisamide administration group compared to those of the control group (Figure 22, p=0.029; Figure 23, p=0.008). It was suggested that administration of zonisamide not only induced axon elongation but also increase in dendrites in the spinal cord segment distal to the damaged area because MAP2 and Tau are highly expressed in dendrites and axons of neuronal cells, respectively. The expression level of NGF was also seemed to be increased in the segment distal to the damaged area as observed in MAP2 and Tau (Figure 18, p=0.053).

These results demonstrated that the expression levels of genes associated with neurite outgrowth were increased in the segment distal to the damaged area.

### Industrial Applicability

Zonisamide or an alkali metal salt thereof according to the present invention is extremely useful for peripheral neuropathy and spinal cord injury. Zonisamide or an alkali metal salt thereof according to the present invention treats "peripheral neuropathy" by regenerating neuronal cells and thus is useful for treating motor dysfunction, sensory dysfunction, and/or autonomic dysfunction associated with "peripheral neuropathy". Moreover, zonisamide or an alkali metal salt thereof according to the present invention treats "spinal cord injury" and is therefore useful for treating motor dysfunction, sensory dysfunction, and/or autonomic dysfunction associated with "spinal cord injury".

## Claims

1. A pharmaceutical composition for treating and/or preventing peripheral neuropathy comprising zonisamide or an alkali metal salt thereof as an active ingredient.

2. The pharmaceutical composition according to claim 1, wherein the peripheral neuropathy comprises at least one neuropathy selected from motor neuropathy, sensory neuropathy, or autonomic neuropathy.

3. The pharmaceutical composition according to claim 2, wherein the peripheral neuropathy at least comprises motor neuropathy.

4. The pharmaceutical composition according to claim 2 or 3, wherein the motor neuropathy is paralysis, numbness, muscle weakness, hyposthenia, muscle atrophy, disorder of skilled movement, wrist dorsiflexion, ankle dorsiflexion, drop foot, hyporeflexia, or vasomotor symptom.

5. The pharmaceutical composition according to any one of claims 2-4, wherein the motor neuropathy is paralysis or numbness.

6. The pharmaceutical composition according to any one of claims 2-5, wherein the sensory neuropathy is pain, paresthesia, or abnormal vibratory sensation.

7. The pharmaceutical composition according to any one of claims 2-6, wherein the autonomic neuropathy is abnormal sweating, diarrhea, constipation, dizziness, shivering, palpitation, or abnormal heart rate variability.
